(19) Europäisches Patentamt
European Patent Office
Office européen des brevets

(11) Publication number: 0 300 074 A1

(12) EUROPEAN PATENT APPLICATION

(21) Application number: 87110639.9

(22) Date of filing: 22.07.87

(51) Int. Cl.4 C07D 471/04 , C07D 241/08 , //A61K31/495,(C07D471/04, 241:00,221:00)

(43) Date of publication of application: 25.01.89 Bulletin 89/04

(84) Designated Contracting States: AT BE CH DE ES FR GB GR IT LI LU NL SE

(71) Applicant: PENNWALT CORPORATION
Pennwalt Building Three Parkway
Philadelphia Pennsylvania 19102(US)

(72) Inventor: Schmiesing, Richard Jon
387 Quigly Dr.
Scottsville New York 14546(US)

(74) Representative: Kraus, Walter, Dr. et al
Patentanwälte Kraus, Weisert & Partner
Thomas-Wimmer-Ring 15
D-8000 München 22(DE)

(54) **Processes and intermediates useful in the preparation of Cis 1,3,4,6,7 IIb-Hexa-hydro-7-aryl-2H-pyrazino[2,1-a]isoquinolines.**

(57) Process of preparing the cis isomeric form of pyrazino [2,1-a] isoquinolines of formula

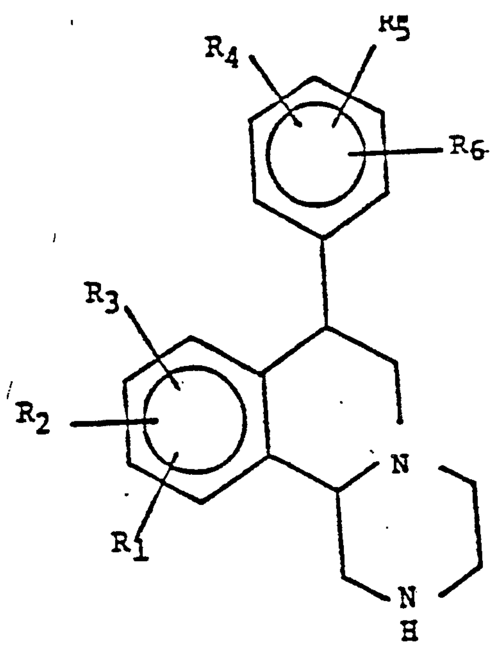

The process involves novel 2-piperazinones of formula

**EP 0 300 074 A1**

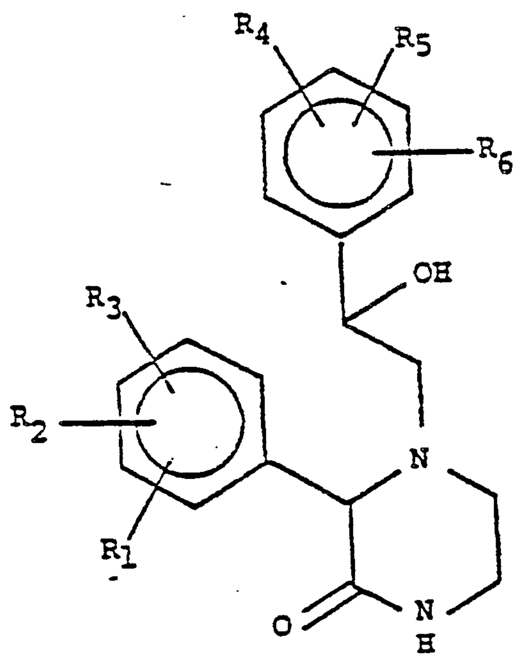

and novel pyrazino [2,1-a] isoquinolines of formula

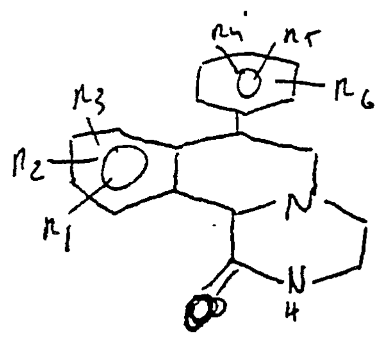

# Processes and Intermediates Useful in the Preparation of Cis 1,3,4,6,7,11b-Hexahydro-7-aryl-2H-pyrazino [ 2,1-a ]isoquinolines (IR 2818)

## Background of the Invention

An efficient process for preparing the cis isomeric form of 1,3,4,6,7,11b-hexahydro-7-aryl-2H-pyrazino [2,1-a]isoquinolines is disclosed. The process involves the use of novel compounds, 3-phenyl-4-(2-hydroxy-2-arylethyl)-2-piperazinones and 3,4,6,7-tetrahydro-7-aryl-2H-pyrazino [2,1-a]isoquinolin-1(11bH)-ones.

## Summary of the Invention

The invention is, in part, the process of making the cis isomeric form of a compound of formula (1)

(1)

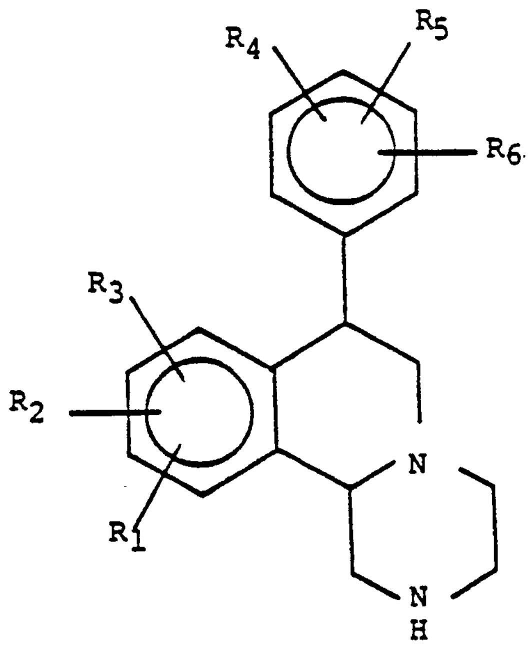

which comprises the steps of

(a) reacting a compound of formula (2)

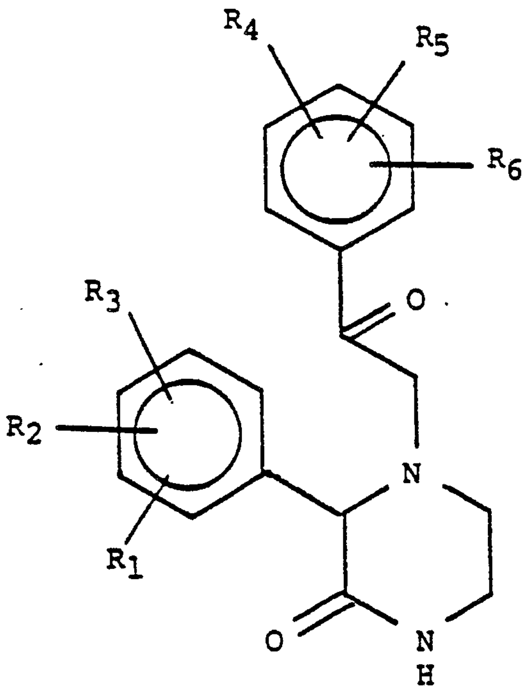

(2)

with a selective reducing agent to form a compound of formula (3)

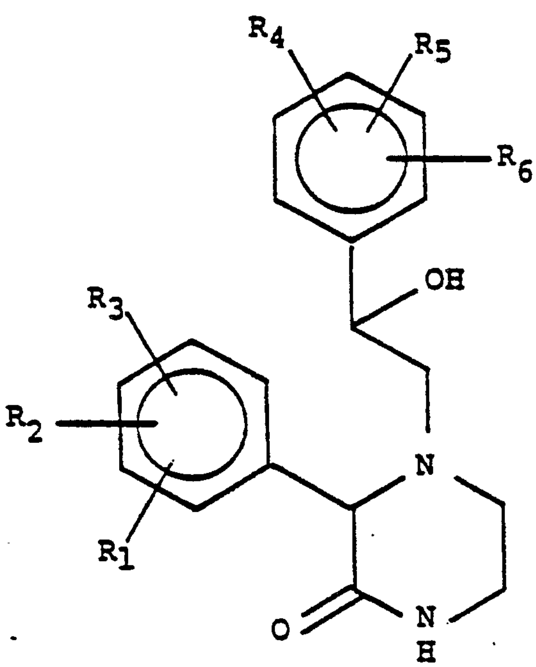

(3)

(b) reacting the compound of formula (3) with a strong acid to form a compound of formula (4)

(4)

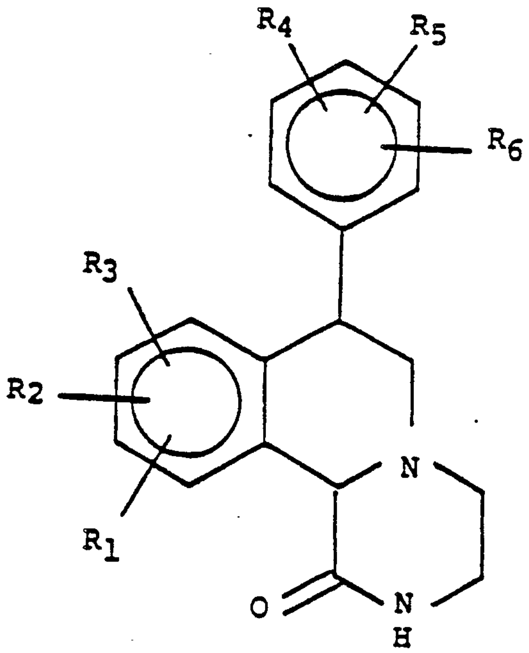

(c) reacting the compound of formula (4) which is either in its trans isomeric form or a mixture of its cis and trans isomeric forms with a base in protic solvent to produce a compound of formula (4) in its cis isomeric form; and

(d) reacting the cis isomeric form of a compound of formula (4) with a reducing agent; wherein in compounds (1), (2), (3) and (4),

$R_1$, $R_2$, $R_3$, $R_4$, $R_5$ and $R_6$ are independently hydrogen, halogen, hydroxy, amino, lower aminoalkyl, trifluoromethyl, lower alkyl, lower alkoxy, lower dialkylamino of lower monoalkylamino.

The invention is additionally each individual step (a), (b), (c) or (d) or the above process.

The invention is also the compounds of formula (3) and the compounds of formula (4).

## Detailed Description of the Invention

### Definitions

"Lower aminoalkyl" is an amino group covalently linked to an alkyl group, straight or branched, containing one to sevel carbon atoms.

"Lower monoalkylamino" is an alkyl group, containing one to seven carbon atoms in a straight or branched chain, linked to an amino moiety.

"Lower dialkylamino" is two alkyl groups, each containing one to seven carbon atoms in a straight or branched chain, linked to an amino moiety.

"Lower alkyl" is an alkyl group containing one to seven carbon atoms in a straight or branched chain.

"Lower alkoxy" is an alkoxy group containing one to seven carbon atoms in a straight or branched chain.

"Halogen" is either chlorine, fluorine, iodine, or bromine.

By the trans isomeric form of either a 1,3,4,6,7,11b-hexahydro-7-aryl-2H-pyrazino [2,1-a] isoquinoline (formula (1)) or a 3,4,6,7-tetrahydro-7-aryl-2H-pyrazino [2,1-a] isoquinolin-1(11bH)-one(formula (4))

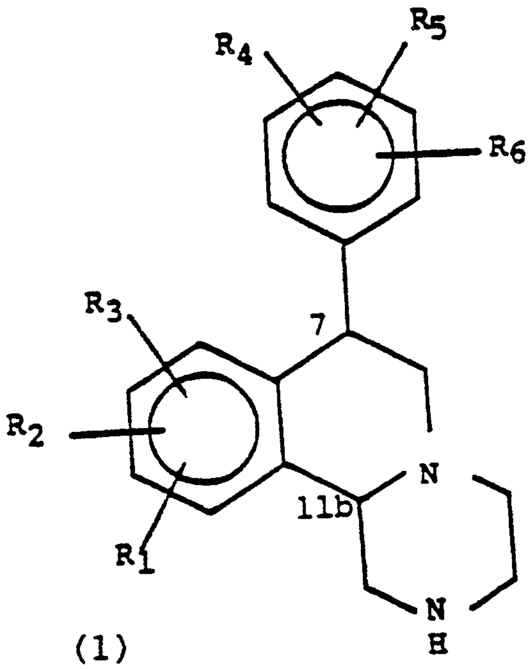

(1)

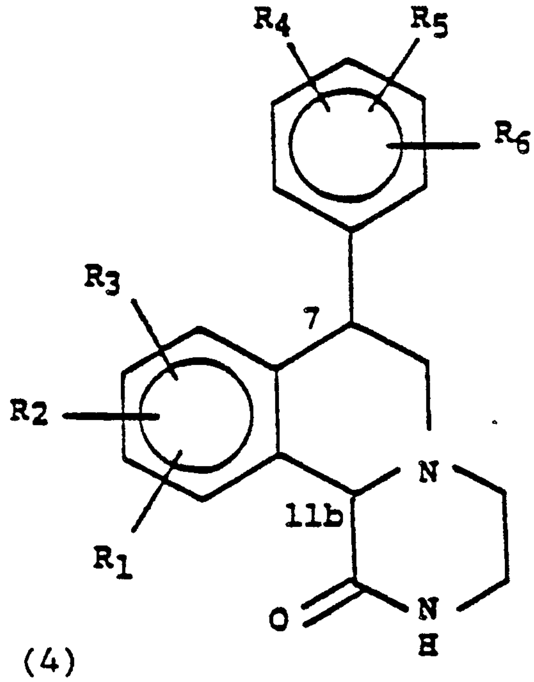

(4)

are meant those forms in which the hydrogen atoms at the 7 and 11b positions of the molecule are on opposite sides of the plane of the ring that contains a single nitrogen atom. In the cis isomeric forms, the hydrogen atoms at the 7 and 11b positions are on the same side of the plane of the ring that contains a single nitrogen atom.

<u>Utility</u>

The compounds of formula (1), in both their cis and trans isomeric forms, are useful because they have antidepressant, anti-histaminic and cholinergic activites. Representative compounds of formula (1) are disclosed and discussed in U.S. Patent No. 4,517,187.

The process of making the cis isomeric form of a compound of formula (1), the individual steps (a), (b), (c), and (d) of that process, and the chemical intermediates [e.g., compounds of formulas (3) and (4)] of that process, are useful in view of the utility of the compounds of formula (1).

Procedures

The processes disclosed in the section, "Summary of the Invention" can be summarized in the following Scheme I:

Scheme I

Procedure (a)

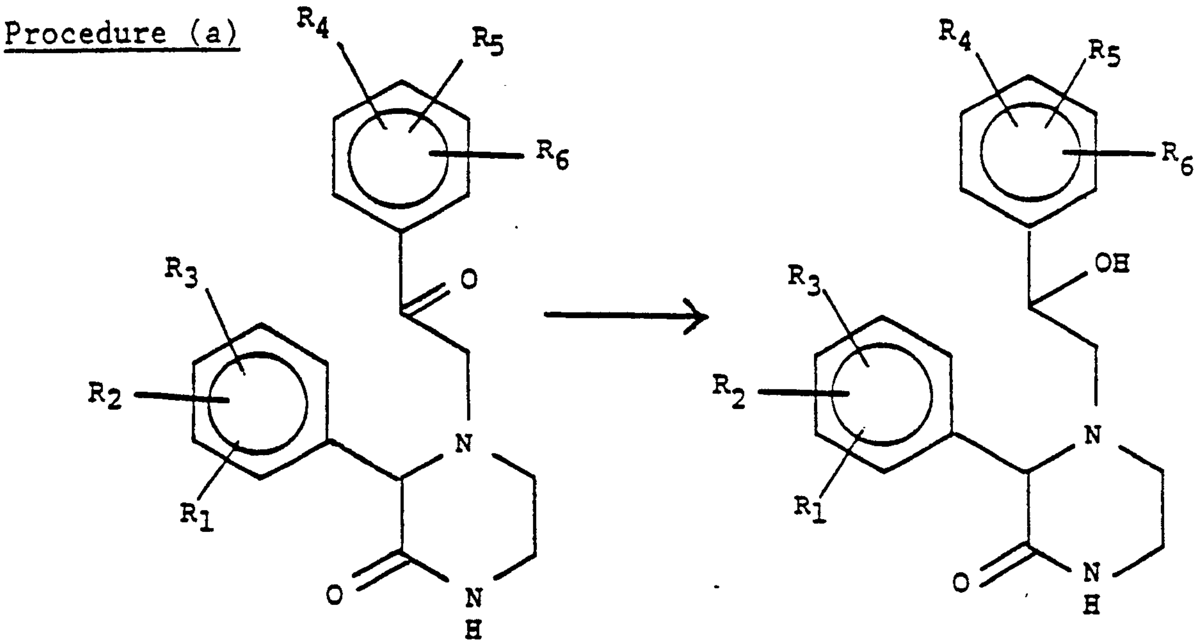

Procedure (b)

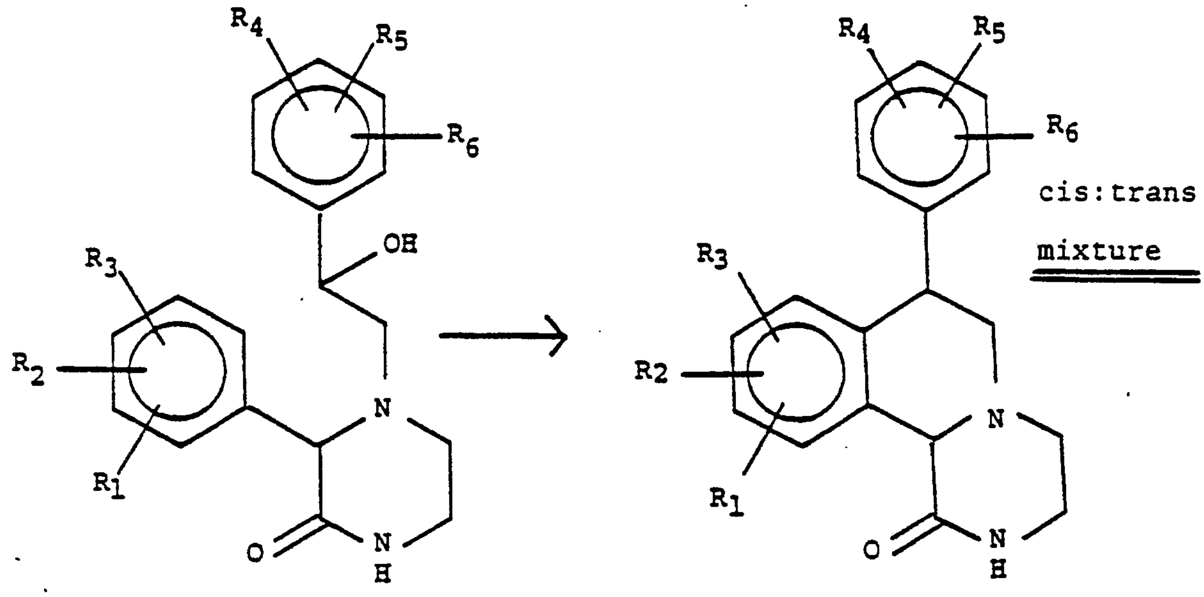


Procedure (c)

5

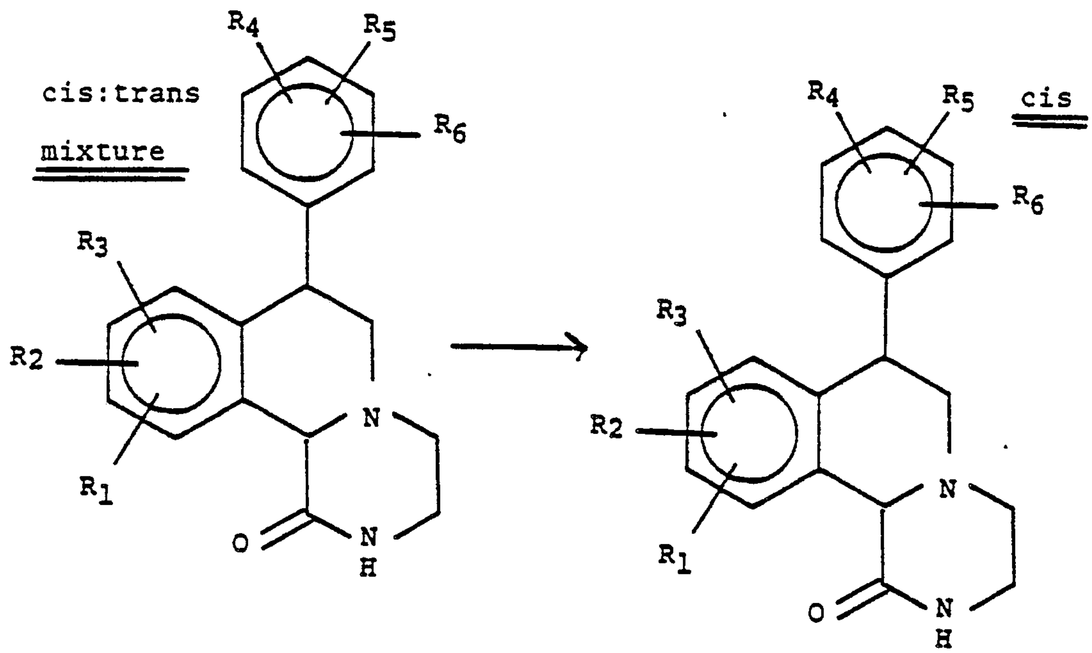

Procedure (d)

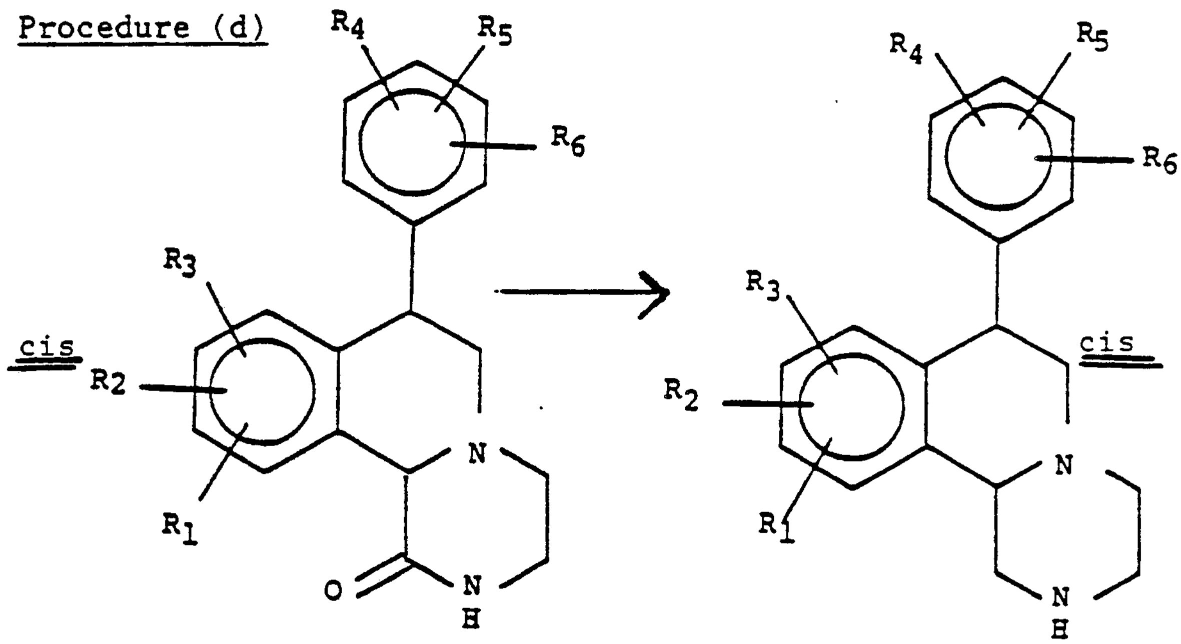

Preparation of 3-phenyl-4-phenacyl-2-piperazinones as starting materials

The starting 3-phenyl-4-phenacyl-2-piperazinones are produced by the following two-step process:

Step (1)

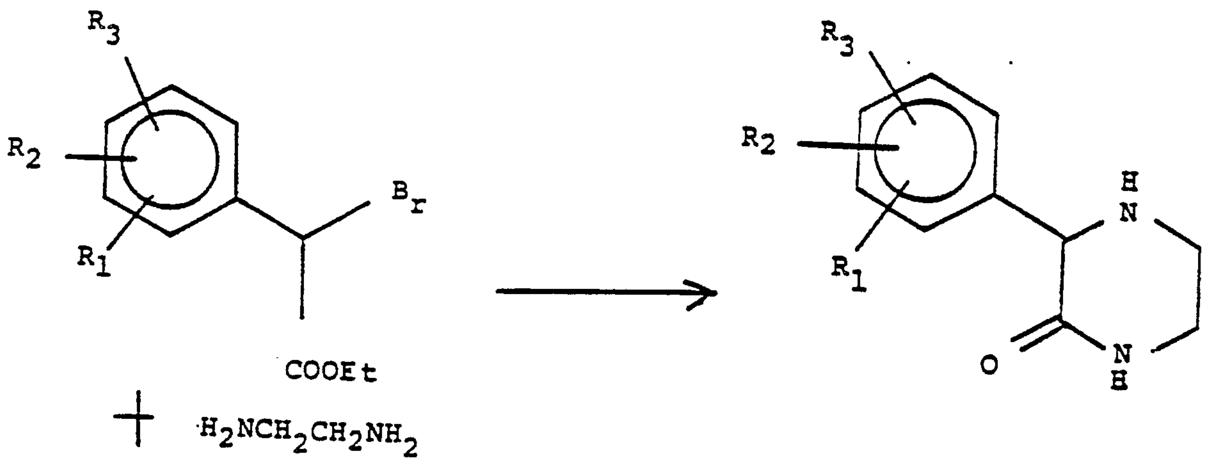

Step (ii)

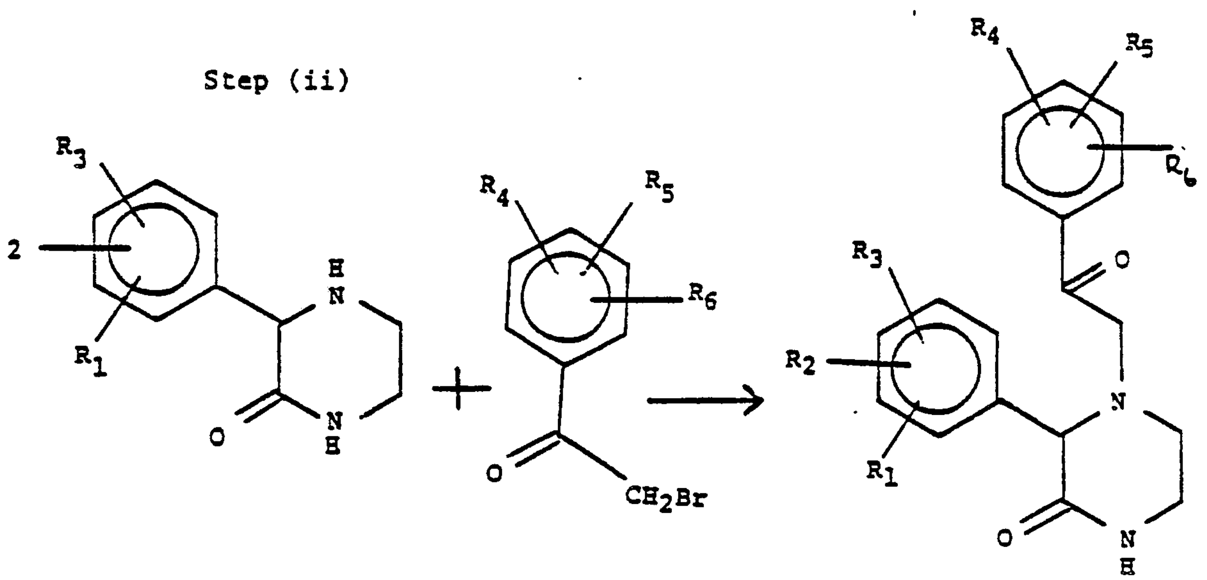

wherein $R_1$, $R_2$, $R_3$,$R_4$,$R_5$ and $R_6$ are independently hydrogen, halogen, hydroxy, protected amino, protected lower aminoalkyl, trifluoromethyl, lower alkyl, lower alkoxy, lower dialkylamino or protected lower monoalkylamino.

When one or more of $R_1$,$R_2$,$R_3$,$R_4$,$R_5$ and $R_6$ are amino, lower aminoalkyl, or lower monoalkylamino, suitable protecting groups are pthalimido, ethoxycarbonyl, benzyloxycarbonyl, and benzyl. After Step (ii) has been completed, the protecting groups are readily removed by techniques such as hydrazinolysis, acid hydrolysis, or hydrogenolysis.

Step (1) is performed in essentially the same manner as reported in the literature

[W.R.Roderick,H.J.Platte, and C.B.Pollard; J.Med.Chem.,9, 181(1966)]. This step allows for the use of ring-substituted ethyl α-bromophenylacetates, substitution which can be retained or chemically altered during the sequence of Scheme I. This particular advantage additionally implies the ready access to Formula I compounds regardless of whether $R_1$,$R_2$,$R_3$,$R_4$,$R_5$ and $R_6$ are the same or different (see also Step ii).

Step (ii) of the process involves alkylation of Step (i) product with an equimolar amount of phenacyl halide in the presence of base. The base can either be an organic tertiary amine such as triethylamine or pyridine, or it can be an inorganic base such as an alkali carbonate or bicarbonate. A particularly useful base is potassium carbonate. The reaction is carried out at from 0° C to 80° C for 3-24 hours in an inert solvent such as chloroform, methylene chloride, dime;thylformamide, acetone, toluene, or a lower alcohol, the halocarbon solvents being particularly convenient. The product is simply isolated by filtration of the reaction mixture and concentration of the filtrate. The product thus obtained is suitable for use in the next reaction without further purification, although pure product may be obtained by recrystallization from organic solvents. Step (ii) allows for the use of a wide variety of readily available ring-substituted phenacyl halides as alkylating agents for analog preparation.

Procedure (a) of Scheme I

This procedure involves reacting a 3-phenyl-4-phenacyl-2-piperazinone with a reducing agent that will reduce the phenyl ketone but not the amide carbonyl of the 3-phenyl-4-phenacyl-2-piperazinone. Reducing agents that will selectively reduce the phenyl ketone are borohydrides, (especially sodium borohydrides), tri-hydrides (especially tri-n-butyl-tin hydride), and hydrogen over a ruthenium (preferably 5%) on carbon catalyst. The reaction is carried out in an inert solvent in which both the reducing agent and the 3-phenyl-4-phenacyl-2-piperazinone are soluble. Suitable solvents include: lower alcohols such as methanol, ethanol or isopropanol for sodium borohydride; hexane, pentane, benzene, toluene and tetrahydrofuran for tri-n-butyl-tin hydride, and water or aqueous ethanol for hydrogen with ruthenium on carbon catalyst. A particularly useful agent is sodium borohydride in a lower alcohol reaction medium such as methanol, ethanol, or isopropanol at from 0° C to 50° C for 1-12 hours. The solid product, 3-phenyl-4-[2-hydroxy-2-(4-chlorophenyl) ethyl]-2-piperazinone for example, is isolated in the usual fashion and is routinely of sufficient purity to be used in the next reaction.

Procedure (b) of Scheme I

Procedure (b) of the process involves a Friedel-Crafts type cyclization of the Procedure (a) product with a suitably strong acid; i.e., one strong enough to accomplish the cyclization. Preferred are suitably strong Bronsted acids, especially polyphosphoric acid or sulphuric acid, and suitably strong Lewis acids. The reaction can take place either in the acid alone or in an inert solvent such as the aromatic hydrocarbons or aromatic halocarbons. For example, reaction in polyphosphoric acid at from 90° C to 130° C for several hours or in concentrated sulfuric acid from 0° C to 30° C for 2-10 hours may be employed, the latter being particularly useful. The solid product [3,4,6,7-tetrahydro-7-(4-chlorophenyl) -2H-pyrazino [2,1-a]-isoquinolin-1(11bH)-one, for example], is isolated by basification of the cooled reaction mixture with aqueous hydroxide followed either by filtration of the solid or by extraction with a suitable organic solvent such as the halocarbons dichloromethane or chloroform; it is enriched as to its trans isomeric form. In preferred embodiments of the reaction, yields greater than 95% can be obtained. The product is suitable for use in the subsequent step without further purification.

Procedure (c) of Scheme I

This procedure involves reaction of Procedure (b) product with a base that is strong enough to cause equilibration of the trans isomeric form of the Procedure (b) product to its cis isomeric form (through a deprotonization reaction followed by a reprotonization reaction). Protic solvents are used with the base. Preferred bases are provided by a lower alcohol solution of the corresponding metal alcoholate; soldium methoxide in methanol is particularly useful. Although the equilibration may be conducted at ambient temperature by stirring in excess of 24 hours, in some cases shorter reaction times (12-24 hours) are

experienced when the equilibration is carried out at reflux temperatures. The cis-enriched product (>100:1 ratio) is conveniently isolated by filtration of the cooled reaction contents and washing the collected white solid with cold methanol. The yield of equilibrated product, cis-3,4,6, 7-tetrahydro-7-(4-chlorophenyl)-2H-pyrazino (2,1-a] isoquinolin-1(11bH)-one for example, in the preferred embodiments is greater than 95%.

Procedure (d) of Scheme I

This step involves reacting the product of Procedure (c) with a reducing agent that will reduce the amide carbonyl of that product. Appropriate reducing agents are aluminum hydrides and boron hydrides, diborane being preferred. The reaction is carried out in an inert solvent in which both the reducing agent and the product of Procedure (c) are soluble. Suitable solvents include dioxane, diethyl ether, dimethoxyethane, or tetrahydrofuran, the latter being particularly useful. The product is isolated directly from the reaction mixture as its dihydrochloride salt upon decomposition of the amine borate complex with aqueous hydrochloric acid. The solid product, cis-1,3,4,6,7,11b-hexahydro-7-(4-chlorophenyl)-2H-pyrazino[2,1-a] isoquinoline dihydrochloride for example, is collected by filtration and is of suitable purity to be used in subsequent reactions.

## Examples

The following examples are specific embodiments of the invention and are not limiting in any way.

## Example 1

### Preparation of 3-Phenyl-4-[2-hydroxy-2-(4-chlorophenyl)ethyl]-2-piperazinone

#### 3-Phenyl-2-piperazinone

To a stirred solution of 1928 g (32.1 mol) of ethylenediamine in 2 liters of absolute ethanol was added a solution of 3130 g (12.8 mol) of ethyl α-bromophenylacetate in 4 liters of absolute ethanol at such a rate so as to maintain reflux (~45. minutes addition time). The mixture was allowed to cool to ambient temperature and then concentrated to a thick yellow slurry. The slurry was stirred with 2 liters of chloroform, filtered, and the solid washed thoroughly with chloroform (total of 8 liters). The filtrate was concentrated to near dryness and the resulting residue stirred for a short time with 2 liters of isopropanol. The solid was collected by filtration, washed with 4 liters of isopropanol, and dried to give 1224g (54 %) of 3-phenyl-2-piperazinone. This material was used as such in the next step. [Pure material could be obtained by recrystallization from benzene and then from acetone to give a white solid (mp. 139.0 - 139.5 °C).]

#### 3-Phenyl-4-(4-chlorophenacyl)-2-piperazinone.

A stirred mixture of 58.0 g (0.33 mol) of 3-phenyl-2-piperazinone, 85 g (0.62 mol) of blended potassium carbonate, and 76.9g (0.33 mol) of 4-chlorophenacyl bromide in 400 ml of dichloromethane was heated at reflux for 12 hours. The mixture was cooled to ambient temperature, filtered, and the solid washed thoroughly with dichloromethane. The filtrate was concentrated to dryness, isopropanol added and stirred, the solid collected by filtration, washed thoroughly with isopropanol and dried in a vacuum oven at 50C to give 92.5g (85 %) of 3-phenyl-4-(4-chlorophenyl)-2-piperazinone. This material was used as such in the next step. [Pure material could be obtained by recrystallization from methanol to give a white solid (mp. 157-158 °C dec.).]

By following essentially the same procedure but substituting phenacyl bromide, 3-chlorophenacyl bromide, 3-hydroxyphenacyl bromide, 2-pthalimidophenacyl bromide, 2-(phthalimidomethyl)phenacyl bromide, 4-(trifluoromethyl)phenacyl bromide, 4-methylphenacyl bromide, 2-methoxyphenacyl bromide, 3-(N-ethyl-N-methylamino)phenacyl bromide, and 4-(N-methyl-N-benzylamino) phenacyl bromide for the 4-chlorophenacyl bromide above results in the formation of the following compounds respectively:
3-phenyl-4-phenacyl-2-piperazinone (mp. 178-180.5 °C dec),
3-phenyl-4-(3-chlorophenacyl)-2-piperazinone (mp. 152-154.5 °C dec), 3-phenyl-4-(3-hydroxyphenacyl)-2-piperazinone,
3-phenyl-4-(2-pthalimidophenacyl)-2-piperazinone,
3-phenyl-4-[2-(pthalimidomethyl)phenacyl]-2-piperazinone,
3-phenyl-4-[4-(trifluoromethyl)phenacyl]-2-piperazinone,
3-phenyl-4-(4-methylphenacyl)-2-piperazinone,
3-phenyl-4-(2-methoxyphenacyl)-2-piperazinone,
3-phenyl-4-[3-(N-ethyl-N-methylamino)phenacyl]-2-piperazinone, and
3-phenyl-4-[4-(N-methyl-N-benzylamino)phenacyl]-2-piperazinone.

By following essentially the same procedure but substituting
3-(2-chlorophenyl)-2-piperazinone,
3-(3-hydroxyphenyl)-2-piperazinone,
3-(2-pthalimidophenyl)-2-piperazinone,
3-[3-(pthalimidomethyl)phenyl]-2-piperazinone,
3-[4-(trifluoromethyl)phenyl]-2-piperazinone,
3-(2-methylphenyl)-2-piperazinone,
3-(3-methoxyphenyl)-2-piperazinone,
3-[4-(N-methyl-N-ethylamino)phenyl]-2-piperazinone, and
3-[2-(methyl-N-benzylamino)phenyl]-2-piperazinone,
for 3-phenyl-2-piperazinone, and phenacyl bromide for 4-chlorophenacyl bromide, results in the formation of the following compounds, respectively:
3-(2-chlorophenyl)-4-phenacyl-2-piperazinone,
3-(3-hydroxyphenyl)-4-phenacyl-2-piperazinone,
3-(2-pthalimidophenyl)-4-phenacyl-2-piperazinone,
3-[3-(pthalimidomethyl)phenyl]-4-phenacyl-2-piperazinone,
3-[4-trifluoromethyl)phenyl]-4-phenacyl-2-piperazinone,
3-(2-methylphenyl)-4-phenacyl-2-piperazinone,
3-(3-methoxyphenyl)-4-phenacyl-2-piperazinone,
3-[4-(N-methyl-N-ethylamino)phenyl]-4-phenacyl-2-piperazinone, and
3-[2-(N-methyl-N-benzylamino)phenyl]-4-phenacyl-2-piperazinone.

3-Phenyl-4-[2-hydroxy-2-(4-chlorophenyl)ethyl]-2-piperazinone.

To a stirred solution of 1066.9 g (3.245 mol) of 3-phenyl-4-(4-chlorophenacyl)-2-piperazinone in 4.2 liters of methanol was added in portions so as to maintain a reaction temperature at or below 50 °C, 62.5g (1.65 mol) of sodium borohydride (30 minutes addition time). The resulting mixture was stirred for an additional 1 hour and concentrated to a yellow amorphous solid. This material was used as such in the next step.

By following essentially the same procedure but substituting
3-phenyl-4-phenacyl-2-piperazinone,
3-phenyl-4-(3-chlorophenacyl)-2-piperazinone,
3-phenyl-4-(3-hydroxyphenacyl)-2-piperazinone,
3-phenyl-4-(2-aminophenacyl)-2-piperazinone,
3-phenyl-4-[2-(aminomethyl)phenacyl]-2-piperazinone,
3-phenyl-4-[4-(rifluoromethyl)phenacyl]-2-piperazinone,
3-phenyl-4-(4-methylphenacyl)-2-piperazinone,
3-phenyl-4-(2-methoxyphenacyl)-2-piperazinone,
3-phenyl-4-[3-(N-ethyl-N-methylamino)phenacyl-2-piperazinone, and
3-phenyl-4-[4-(N-methylamino)phenacyl-2-piperazinone, for the
3-phenyl-4-(4-chlorophenacyl)-2-piperazinone results in the formation of the following compounds respectively,
3-phenyl-4-(2-hydroxy-2-phenylethyl)-2-piperazinone,
3-phenyl-4-[2-hydroxy-2-(3-chlorophenyl)ethyl]-2-piperazinone,
3-phenyl-4-[2-hydroxy-2-(3-hydroxyphenyl)ethyl]-2-piperazinone,

3-phenyl-4-[2-hydroxy-2-(2-aminophenyl)ethyl-2-piperazinone,
3-phenyl-4-[2-hydroxy-2-[2-(aminomethyl)phenyl]ethyl]-2-piperazinone,
3-phenyl-4-[2-hydroxy-2-[3-(trifluoromethyl)phenyl]ethyl]-2-piperazinone,
3-phenyl-4-[2-hydroxy-2-(4-methylphenyl)ethyl]-2-piperazinone,
3-phenyl-4-[2-hydroxy-2-(2-methoxyphenyl)ethyl]-2-piperazinone,
3-phenyl-4-[2-hydroxy-2-[3-(N-ethyl-N-methylamino)ethyl]-2-piperazinone, and
3-phenyl-4-[2-hydroxy-2-[4-(N-methylamino)ethyl]-2-piperazinone.

By following essentially the same procedure but substituting
3-(2-chlorophenyl)-4-phenacyl-2-piperazinone,
3-(3-hydroxyphenyl)-4-phenacyl-2-piperazinone,
3-(2-aminophenyl)-4-phenacyl-2-piperazinone,
3-[3-aminomethyl)phenyl]-4-phenacyl-2-piperazinone,
3-[4-(trifluoromethyl)phenyl]-4-phenacyl-2-piperazinone,
3-(2-methylphenyl)-4-phenacyl-2-piperazinone,
3-(3-methoxyphenyl)-4-phenacyl-2-piperazinone,
3-[4-(N-methyl-N-ethylamino)phenyl]-4-phenacyl-2-piperazinone, and
3-[2-(methylamino)phenyl]-4-phenacyl-2-piperazinone for 3-phenyl-4-phenacyl-2-piperazinone, results in the formation of the following compounds, respectively:
3-(2-chlorophenyl)-4-(2-hydroxy-2-phenylethyl)-2-piperazinone,
3-(3-hydroxyphenyl)-4-(2-hydroxy-2-phenylethyl)-2-piperazinone,
3-(2-aminophenyl)-4-(2-hydroxy-2-phenylethyl)-2-piperazinone,
3-[3-(aminomethyl)phenyl]-4-(2-hydroxy-2-phenylethyl)-2-piperazinone,
3-[4-(trifluoromethyl)phenyl]-4-(2-hydroxy-2-phenylethyl)-2-piperazinone,
3-(2-methylphenyl)-4-(2-hydroxy-2-phenylethyl)-2-piperazinone,
3-(3-methoxyphenyl)-4-(2-hydroxy-2-phenylethyl)-2-piperazinone,
3-[4-(N-methyl-N-ethylamino)phenyl]-4-(2-hydroxy-2-phenylethyl)-2-piperazinone, and
3-[2-(methylamino)phenyl]-4-(2-hydroxy-2-phenylethyl)-2-piperazinone.

## Example 2

### Preparation of 3,4,6,7-tetrahydro-7-(4-chlorophenyl)-2H-pyrazino-[2,1-a]isoquinolin-1(11bH)-one.

To the ice-cooled yellow amorphous solid 3-phenyl-4-(2-hydroxy-2-(4-chlorophenyl)ethyl]-2-piperazinone obtained from the previous step of the process was added in a rapid stream 4.3 liters of concentrated sulfuric acid. The resulting mixture was stirred at ambient temperature overnight and then poured onto a mixture of 90 kg of ice and 11.2 liters of 50% aqueous sodium hydroxide. The solid was collected by filtration, washed neutral with water, and dried in a vacuum oven at 50 °C to give 997.1g (98%) of 3,4,6,7-tetrahydro-7-(4-chlorophenyl)-2H-pyrazino [2,1-a] isoquinolin-1(11bH)-one as a pale yellow solid consisting of a 4.5:1 trans:cis mixture of isomers. This material was used as such in the next step of the process.

By following essentially the same procedure but substituting
3-phenyl-4-(2-hydroxy-2-phenylethyl)-2-piperazinone,
3-phenyl-4-[2-hydroxy-2-(3-chlorophenyl)ethyl]-2-piperazinone,
3-phenyl-4-[2-hydroxy-2-(3-hydroxyphenyl)ethyl]-2-piperazinone,
3-phenyl-4-[2-hydroxy-2-(2-aminophenyl)ethyl]-2-piperazinone,
3-phenyl-4-[2-hydroxy-2-[2-(aminoethyl)phenyl]ethyl]-2-piperazinone,
3-phenyl-4-[2-hydroxy-2-[3-(trifluoromethyl)phenyl]ethyl]-2-piperazinone,
3-phenyl-4-[2-hydroxy-2-(4-methylphenyl)ethyl]-2-piperazinone,
3-phenyl-4-[2-hydroxy-2-(2-methoxyphenyl)ethyl]-2-piperazinone,
3-phenyl-4-[2-hydroxy-2-[3-(N-ethyl-N-methylamino)phenyl]ethyl]-2-piperazinone, and
3-phenyl-4-[2-hydroxy-2-[4-(N-methylamino)phenyl]ethyl]-2-piperazinone for the
3-phenyl-4-[2-hydroxy-2-(4-chlorophenyl)ethyl]-2-piperazinone results in the formation of the following compounds respectively,
3,4,6,7-tetrahydro-7-phenyl-2H-pyrazino [2,1-a]isoquinolin-1(11bH) -one,
3,4,6,7-tetrahydro-7-(3-chlorophenyl)-2H-pyrazino-[2,1-a]isoquinolin-1(11bH)-one,

3,4,6,7-tetrahydro-7-(3-hydroxyphenyl)-2H-pyrazino[2,1-a]isoquinolin-1(11bH)-one,
3,4,6,7-tetrahydro-7-(2-aminophenyl)-2H-pyrazino-[2,1-a]isoquinolin-1(11bH)-one,
3,4,6,7-tetrahydro-7-[2-(aminomethyl)phenyl]-2H-pyrazino[2,1-a]isoquinolin-1(11bH)-one,
3,4,6,7-tetrahydro-7-[3-(trifluoromethyl)phenyl]-2H-pyrazino[2,1-a]isoquinolin-1(11bH)-one,
3,4,6,7-tetrahydro-7-(4-methylphenyl)-2H-pyrazino-[2,1-a]isoquinolin-1(11bH)-one,
3,4,6,7-tetrahydro-7-(2-methoxyphenyl)-2H-pyrazino-[2,1-a]isoquinolin-1(11bH)-one,
3,4,6,7-tetrahydro-7-[3-(N-ethyl-N-methylamino)phenyl]-2H-pyrazino-[2,1-a]isoquinolin-1(11bH)-one, and
3,4,6,7-tetrahydro-7-[4-(N-methylamino)phenyl]-2H-pyrazino[2,1-a]isoquinolin-1(11bH)-one.

Following the same procedure but substituting 3-(2-chlorophenyl)-4-(2-hydroxy-2-phenylethyl)-2-piperazinone,
3-(3-hydroxyphenyl)-4-(2-hydroxy-2-phenylethyl)-2-piperazinone,
3-(2-aminophenyl)-4-(2-hydroxy-2-phenylethyl)-2-piperazinone,
3-[3-(aminomethyl)phenyl]-4-(2-hydroxy-2-phenylethyl)-2-piperazinone,
3-[4-(trifluoromethyl)phenyl]-4-(2-hydroxy-2-phenylethyl)-2-piperazinone,
3-(2-methylphenyl)-4-(2-hydroxy-2-phenylethyl)-2-piperazinone,
3-(3-methoxyphenyl)-4-(2-hydroxy-2-phenylethyl)-2-piperazinone,
3-[4-(N-methyl-N-ethylamino)phenyl]-4-(2-hydroxy-2-phenylethyl)-2-piperazinone,
3-[2-(methylamino)phenyl]-4-(2-hydroxy-2-phenylethyl)-2-piperazinone,
for the 3-phenyl-4-[2-hydroxy-2-(4-chlorophenyl)ethyl]-2-piperazinone dihydrochloride results in the formation of the following compounds, respectively:
3,4,6,7-tetrahydro-7-phenyl-11-chloro-2H-pyrazino[2,1-a]isoquinolin-1(11bH)-one dihydrochloride,
3,4,6,7-tetrahydro-7-phenyl-8-hydroxy-2H-pyrazino[2,1-a]isoquinolin-1(11bH)-one dihydrochloride, and
3,4,6,7-tetrahydro-7-phenyl-10-hydroxy-2H-pyrazino[2,1-a]isoquinolin-1(11bH)-one dihydrochloride,
3,4,6,7-tetrahydro-7-phenyl-11-amino-2H-pyrazino[2,1-a]isoquinolin-1(11bH)-one dihydrochloride,
3,4,6,7-tetrahydro-7-phenyl-8-aminomethyl-2H- pyrazino[2,1-a]isoquinolin-1(11bH)-one dihydrochloride, and
3,4,6,7-tetrahydro-7-phenyl-10-aminomethyl-2H-pyrazino[2,1-a]isoquinolin-1(11bH)-one dihydrochloride,
3,4,6,7-tetrahydro-7-phenyl-9-trifluoromethyl-2H-pyrazino[2,1-a]isoquinolin-1(11bH)-one dihydrochloride,
3,4,6,7-tetrahydro-7-phenyl-11-methyl-2H-pyrazino[2,1-a]isoquinolin-1(11bH)-one dihydrochloride,
3,4,6,7-tetrahydro-7-phenyl-8-methoxy-2H-pyrazino[2,1-a]isoquinolin-1(11bH)-one dihydrochloride and
3,4,6,7-tetrahydro-7-phenyl-10-methoxy-2H-pyrazino[2,1-a]isoquinolin-1(11bH)-one dihydrochloride,
3,4,6,7-tetrahydro-7-phenyl-9-(N-methyl-N-ethylamino)-2H-pyrazino[2,1-a]isoquinolin-1(11bH)-one dihydrochloride,
and
3,4,6,7-tetrahydro-7-phenyl-11-methylamino-2H-pyrazino[2,1-a]isoquinolin-1(11bH)-one dihydrochloride.
The mixtures of 8-position and 10-position substituted compounds can be separated into their individual components by common techniques such as selective crystallization or chromatography (for example, silica gel chromatography).

## Example 3

### Preparation of cis-3,4,6,7-tetrahydro-7-(4-chlorophenyl)-2H-pyrazino [2,1-a] isoquinolin-1(11bH)-one.

To 1905 g (6.09 mol) of a mixture of the cis and trans isomeric forms of 3,4,6,7-tetrahydro-7-(4-chlorophenyl-2H-pyrazino[2,1-a]isoquinolin-1(11bH)-one (trans:cis ratio equal to 4.5:1) in 4 liters of methanol was added, while stirring at ambient temperature, a solution of 36.5g (1.59 mol) of metallic sodium in 15 liters of methanol. The resulting stirred suspension was heated at reflux for 24 hours, allowed to cool at ambient temperature, and stirred for a further 12 hours. The solid was collected by filtration, washed thoroughly with methanol, and dried in a vacuum oven at 60 °C to give 1692 g (89%) of cis-3,4,6,7-tetrahydro-7-(4-chlorophenyl)-2H-pyrazino [2,1-a]isoquinolin-1(11bH)-one as a white solid (mp. 239-244 °C dec.) contaminated by less than 1% of the trans isomer. this material was used as such in the next step of the process.

Similarly, by following essentially the same procedure but substituting a mixture of the cis and trans forms of 3,4,6,7-tetrahydro-7-phenyl-2H-pyrazino[2,1-a]isoquinolin-1(11bH)-one,
3,4,6,7-tetrahydro-7-(3-hydroxyphenyl)-2H-pyrazino[2,1-a]isoquinolin-1(11bH)-one,
3,4,6,7-tetrahydro-7-(2-aminophenyl)-2H-pyrazino[2,1-a]isoquinolin-1(11bH)-one,

3,4,6,7-tetrahydro-7-(2-(aminomethyl)phenyl]-2H-pyrazino[2,1-a]isoquinolin-1(11bH)-one,
3,4,6,7-tetrahydro-7-[3-(trifluoromethyl)phenyl]-2H-pyrazino[2,1-a]isoquinolin-1(11bH)-one,
3,4,6,7-tetrahydro-7-(2-methoxymethyl)-2H-pyrazino[2,1-a]isoquinolin-1(11bH)-one
3,4,6,7-tetrahydro-7-[3-(N-methyl-N-ethylamino)phenyl]-2H-pyrazino[2,1-a]isoquinolin-1(11bH)-one,
3,4,6,7-tetrahydro-7-[4-(N-methylamino)phenyl]-2H-pyrazino[2,1-a]isoquinolin-1(11bH)-one,
3,4,6,7-tetrahydro-7-[4-(N-methylamino)phenyl]-2H-pyrazino[2,1-a]isoquinolin-1(11bH)-one,
3,4,6,7-tetrahydro-7-[4-(N-methylphenyl)-2H-pyrazino [2,1-a]isoquinolin-1(11bH)-one,
3,4,6,7-tetrahydro-7-phenyl-11-chloro-2H-pyrazino[2,1-a]isoquinolin-1(11bH)-one dihydrochloride,
3,4,6,7-tetrahydro-7-phenyl-8-hydroxy-2H-pyrazino[2,1-a]isoquinolin-1(11bH)-one dihydrochloride,
3,4,6,7-tetrahydro-7-phenyl-10-hydroxy-2H-pyrazino[2,1-a]isoquinolin-1(11bH)-one dihydrochloride,
3,4,6,7-tetrahydro-7-phenyl-11-amino-2H-pyrazino[2,1-a]isoquinolin-1(11bH)-one dihydrochloride,
3,4,6,7-tetrahydro-7-phenyl-8-aminomethyl-2H-pyrazino[2,1-a]isoquinolin-1(11bH)-one dihydrochloride,
3,4,6,7-tetrahydro-7-phenyl-10-aminomethyl-2H-pyrazino[2,1-a]isoquinolin-1(11bH)-one dihydrochloride,
3,4,6,7-tetrahydro-7-phenyl-9-trifluoromethyl-2H-pyrazino[2,1-a]isoquinolin-1(11bH)-one dihydrochloride,
3,4,6,7-tetrahydro-7-phenyl-11-methyl-2H-pyrazino[2,1-a]isoquinolin-1(11bH)-one dihydrochloride,
3,4,6,7-tetrahydro-7-phenyl-8-methoxy-2H-pyrazino[2,1-a]isoquinolin-1(11bH)-one dihydrochloride,
3,4,6,7-tetrahydro-7-phenyl-10-methoxy-2H-pyrazino[2,1-a]isoquinolin-1(11bH)-one dihydrochloride,
3,4,6,7-tetrahydro-7-phenyl-9-(N-methyl-N-ethylamino)-2H-pyrazino[2,1-a]isoquinolin-1(11bH)-one dihydrochloride,
and
3,4,6,7-tetrahydro-7-phenyl-11-methylamino-2H-pyrazino[2,1-a]isoquinolin-1(11bH)-one dihydrochloride, for the mixture of the cis and trans forms of 3,4,6,7-tetrahydro-7-(4-chlorophenyl)-2H-pyrazino[2,1-a]isoquinolin-1(11bH)-one results in the cis form of the compound substituted for 3,4,6,7-tetrahydro-7-(4-chlorophenyl)-2H-pyrazino[2,1-a]isoquinolin-1(11bH)-one substantially free of the corresponding trans form.

## Example 4

### Preparation of cis-1,3,4,6,7,11b-hexahydro-7-(4-chlorophenyl) -2H-pyrazino [2,1-a] isoquinoline dihydrochloride.

To a stirred suspension of 1810 g (5.79 mol) of cis-3,4,6,7-tetrahydro-7-(4-chlorophenyl)-2H-pyrazino [2,1-a]isoquinolin-1(11bH)-one in 5.7 liters of tetrahydrofuran under nitrogen was added rapidly 28.6 liters of 1.0 M solution of borane in tetrahydrofuran. The mixture was heated at reflux for 15 hours, cooled to ambient temperature, and treated cautiously with 4.2 liters of 2.5 M aqueous hydrochloric acid. This resulting mixture was heated at reflux for 3.5 hours and then allowed to cool to ambient temperature. The solid was collected by filtration, washed thoroughly with tetrahydrofuran, and dried in a vacuum oven at 65 °C to give 2236 g of cis-1,3,4,6,7,11b-hexahydro-7-(4-chlorophenyl)-2H-pyrazino [2,1-a] isoquinoline dihydrochloride contaminated with boric acid.

By following essentially the same procedure but substituting
cis-3,4,6,7-tetrahydro-7-phenyl-2H-pyrazino-[2,1-a] isoquinolin-1(11bH)-one,
cis-3,4,6,7-tetrahydro-7-(3-chlorophenyl)-2H-pyrazino[2,1-a]isoquinolin-1(11bH)-one,
cis-3,4,6,7-tetrahydro-7-(3-hydroxyphenyl)-2H-pyrazino[2,1-a]isoquinolin-1(11bH)-one,
cis-3,4,6,7-tetrahydro-7-(2-aminophenyl)-2H-pyrazino[2,1-a]isoquinolin-1(11bH)-one,
cis-3,4,6,7-tetrahydro-7-[2-(aminomethyl)phenyl]-2H-pyrazino[2,1-a]isoquinolin-1(11bH)-one,
cis-3,4,6,7-tetrahydro-7-[3-(trifluoromethyl)phenyl]-2H-pyrazino-[2,1-a]isoquinolin-1(11bH)-one,
cis-3,4,6,7-tetrahydro-7-(2-methoxyphenyl)-2H-pyrazino[2,1-a]isoquinolin-1(11bH)-one,
cis-3,4,6,7-tetrahydro-7-[3-(N-ethyl-N-methylamino)phenyl]-2H-pyrazino-[2,1-a]isoquinolin-1(11bH)-one,
cis-3,4,6,7-tetrahydro-7-[4-(methylamino)phenyl]-2H-pyrazino-[2,1-a]isoquinolin-1(11bH)-one and
cis-3,4,6,7-tetrahydro-7-(4-methylphenyl)-2H-pyrazino[2,1-a]isoquinolin-1(11bH)-one for the cis-3,4,6,7-tetrahydro-7-(4-chlorophenyl)-2H-pyrazino-[2,1-a]isoquinolin-1(11bH)-one results in the formation of the following compounds respectively,
cis-1,3,4,6,7,11b-hexahydro-7-phenyl-2H-pyrazino-[2,1-a] isoquinoline dihydrochloride,
cis-1,3,4,6,7,11b-hexahydro-7-(3-chlorophenyl)-2H-pyrazino [2,1-a] isoquinoline dihydrochloride,
cis-1,3,4,6,7,11b-hexahydro-7-(3-hydroxyphenyl)-2H-pyrazino [2,1-a] isoquinoline dihydrochloride,
cis-1,3,4,6,7,11b-hexahydro-7-(2-aminophenyl)-2H-pyrazino [2,1-a] isoquinoline dihydrochloride,

cis-1,3,4,6,7,11b-hexahydro-7-[2-(aminomethyl)phenyl]-2H-pyrazino [2,1-a] isoquinoline dihydrochloride,
cis-1,3,4,6,7,11b-hexahydro-7-[3-(trifluoromethyl)phenyl] -2H-pyrazino [2,1-a] isoquinoline dihydrochloride,
cis-1,3,4,6,7,11b-hexahydro-7-(2-methoxyphenyl)-2H-pyrazino[2,1-a] isoquinoline dihydrochloride,
cis-1,3,4,6,7,11b-hexahydro-7-[3-(N-ethyl-N-methylamino) phenyl]-2H-pyrazino-[2,1-a]isoquinoline dihydrochloride,
cis-1,3,4,6,7,11b-hexahydro-7-[4-(N-methylamino)phenyl]-2H-pyrazino [2,1-a]isoquinoline dihydrochloride, and
cis-1,3,4,6,7,11b-hexahydro-7-(4-methylphenyl)-2H-pyrazino[2,1-a] isoquinoline dihydrochloride.

By following essentially the same procedure but substituting
cis-3,4,6,7-tetrahydro-7-phenyl-11-chloro-2H-pyrazino[2,1-a]isoquinolin-1(11bH)-one dihydrochloride,
cis-3,4,6,7-tetrahydro-7-phenyl-8-hydroxy-2H-pyrazino[2,1-a]isoquinolin-1(11bH)-one dihydrochloride,
cis-3,4,6,7-tetrahydro-7-phenyl-10-hydroxy-2H-pyrazino[2,1-a]isoquinolin-1(11bH)-one dihydrochloride,
cis-3,4,6,7-tetrahydro-7-phenyl-11-amino-2H-pyrazino[2,1-a]isoquinolin-1(11bH)-one dihydrochloride,
cis-3,4,6,7-tetrahydro-7-phenyl-8-aminomethyl-2H-pyrazino[2,1-a]isoquinolin-1(11bH)-one dihydrochloride,
cis-3,4,6,7-tetrahydro-7-phenyl-10-aminomethyl-2H-pyrazino[2,1-a]isoquinolin-1(11bH)-one dihydrochloride,
cis-3,4,6,7-tetrahydro-7-phenyl-9-trifluoromethyl-2H-pyrazino[2,1-a]isoquinolin-1(11bH)-one dihydrochloride,
cis-3,4,6,7-tetrahydro-7-phenyl-11-methyl-2H-pyrazino [2,1-a]isoquinolin-1(11bH)-one dihydrochloride,
cis-3,4,6,7-tetrahydro-7-phenyl-8-methoxy-2H-pyrazino[2,1-a]isoquinolin-1(11bH)-one dihydrochloride,
cis-3,4,6,7-tetrahydro-7-phenyl-10-methoxy-2H-pyrazino[2,1-a]isoquinolin-1(11bH)-one dihydrochloride,
cis-3,4,6,7-tetrahydro-7-phenyl-9-(N-methyl-N-ethylamino)-2H-pyrazino[2,1-a]isoquinolin-1(11bH)-one dihydrochloride, and
cis-3,4,6,7-tetrahydro-7-phenyl-11-methylamino-2H-pyrazino[2,1-a]isoquinolin-1(11bH)-one dihydrochloride,
for the cis-3,4,6,7-tetrahydro-7-(4-chlorophenyl)-2H-pyrazino[2,1-a]isoquinolin-1(11bH)-one results in the formation of the following compounds, respectively:
cis-1,3,4,6,7,11b-hexahydro-7-phenyl-11-chloro-2H-pyrazino[2,1-a]isoquinoline dihydrochloride,
cis-1,3,4,6,7,11b-hexahydro-7-phenyl-8-hydroxy-2H-pyrazino[2,1-a]isoquinoline dihydrochloride,
cis-1,3,4,6,7,11b-hexahydro-7-phenyl-10-hydroxy-2H-pyrazino[2,1-a]isoquinoline dihydrochloride,
cis-1,3,4,6,7,11b-hexahydro-7-phenyl-11-amino-2H-pyrazino[2,1-a]isoquinoline dihydrochloride,
cis-1,3,4,6,7,11b-hexahydro-7-phenyl-8-aminomethyl-2H-pyrazino[2,1-a]isoquinoline dihydrochloride,
cis-1,3,4,6,7,11b-hexahydro-7-phenyl-10-aminomethyl-2H-pyrazino[2,1-a]isoquinoline dihydrochloride,
cis-1,3,4,6,7,11b-hexahydro-7-phenyl-9-trifluoromethyl-2H-pyrazino[2,1-a]isoquinoline dihydrochloride,
cis-1,3,4,6,7,11b-hexahydro-7-phenyl-11-methyl-2H-pyrazino[2,1-a]isoquinoline dihydrochloride,
cis-1,3,4,6,7,11b-hexahydro-7-phenyl-8-methoxy-2H-pyrazino[2,1-a]isoquinoline dihydrochloride,
cis-1,3,4,6,7,11b-hexahydro-7-phenyl-10-methoxy-2H-pyrazino[2,1-a]isoquinoline dihydrochloride,
cis-1,3,4,6,7,11b-hexahydro-7-phenyl-9-(N-methyl-N-ethylamino-2H-pyrazino[2,1-a]isoquinoline dihydrochloride,
and
cis-1,3,4,6,7,11b-hexahydro-7-phenyl-11-methylamino-2H-pyrazino[2,1-a]isoquinoline dihydrochloride.

Use of the procedures in Examples 1 to 4, but with an appropriate choice of reagents in which two or more of the $R_1$,$R_2$,$R_3$,$R_4$,$R_4$ and $R_6$ substitution groups are other than hydrogen, results in the corresponding multisubstituted final product.

## Claims

1. The process of making the cis isomeric form of a compound of formula (1)

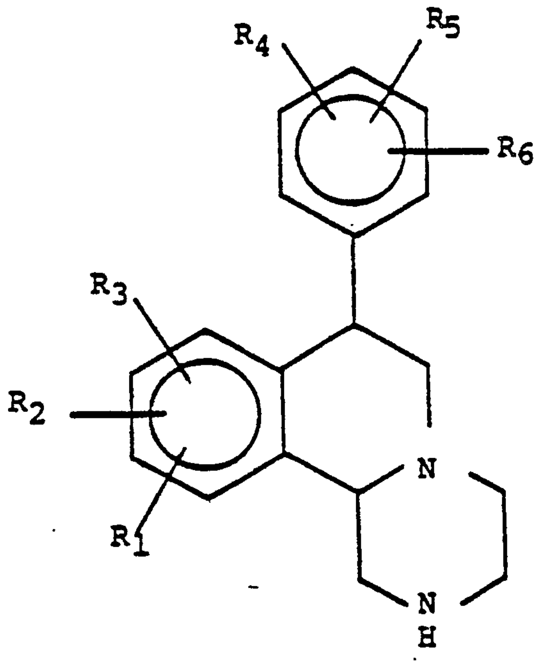

(1)

which comprises the steps of
(a) reacting a compound of formula (2)

(2)

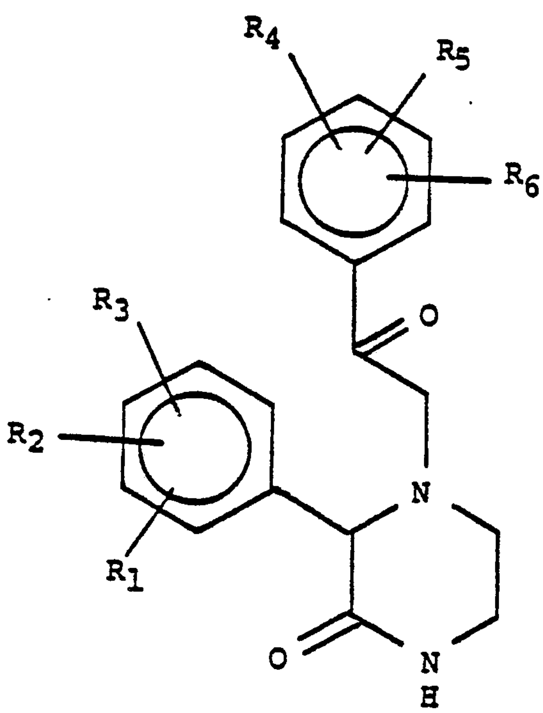

with a selective reducing agent to form a compound of formula (3)

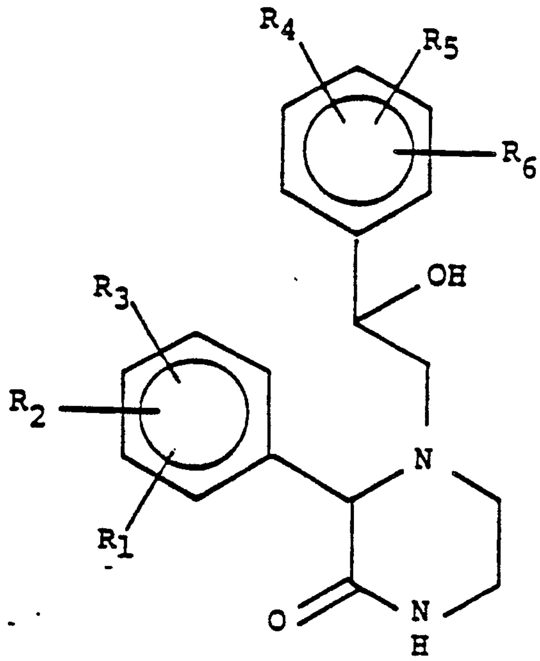

(3)

(b) reacting the compound of formula (3) with a strong acid to form a compound of formula (4)

(4)

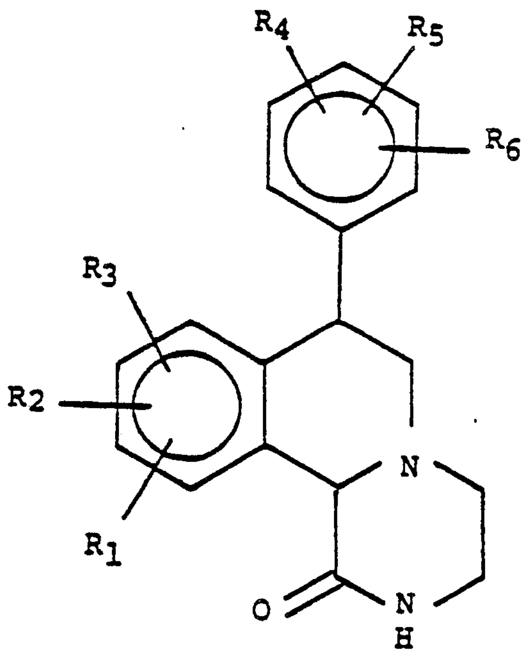

(c) reacting the compound of formula (4) which is either in its trans isomeric form or a mixture of its cis and trans isomeric forms with a base in protic solvent to produce a compound of formula (4) in its cis isomeric form; and

(d) reacting the cis isomeric form of a compound of formula (4) with a reducing agent;

wherein in compounds (1),(2),(3) and (4),

$R_1$, $R_2$, $R_3$, $R_4$, $R_5$ and $R_6$ are independently hydrogen, halogen, hydroxy, amino, lower aminoalkyl, trifluoromethyl, lower alkyl, lower alkoxy, lower dialkylamino or lower monoalkylamino.

2. The process of making the cis isomeric form of the compound of formula (1)

(1)

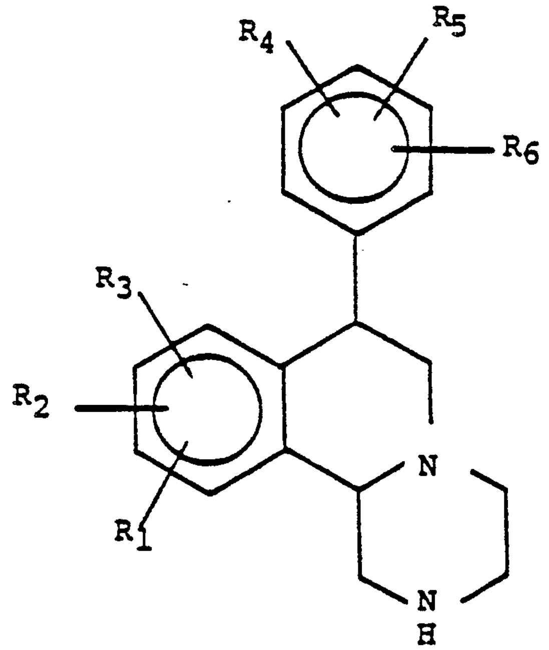

which comprises reacting the cis isomeric form of a compound of formula (4)

(4)

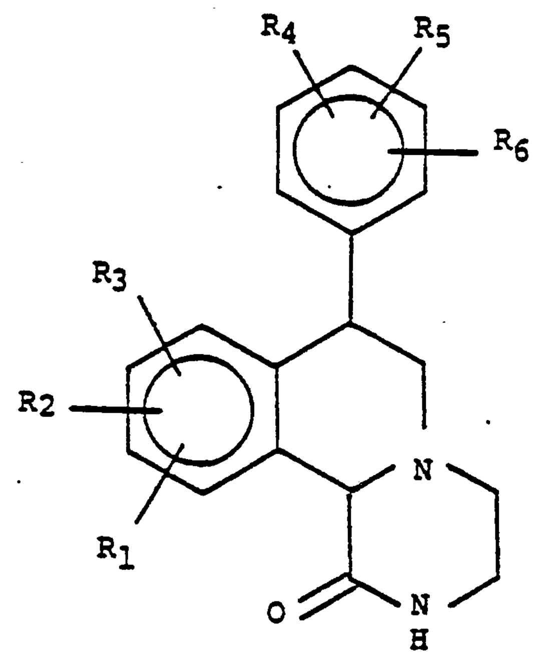

with a reducing agent;
wherein in compounds (1) and (4),
$R_1$, $R_2$, $R_3$, $R_4$, $R_5$ and $R_6$ are independently hydrogen, halogen, hydroxy, amino, lower aminoalkyl, trifluoromethyl, lower alkyl, lower alkoxy, lower dialkylamino or lower monoalkylamino.

3. The process of making the cis isomeric form of a compound of formula (4)

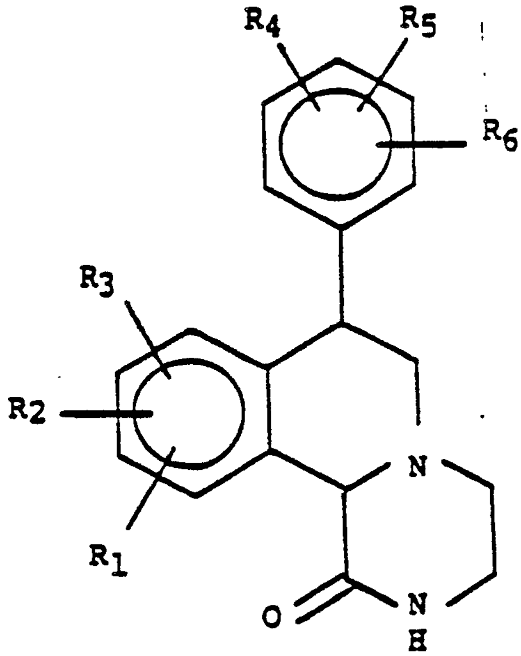

(4)

which comprises a compound of formula (4) that is either in its trans isomeric form or a mixture of its cis and trans isomeric forms with a base in a protic solvent;
wherein,
$R_1$, $R_2$, $R_3$, $R_4$, $R_5$ and $R_6$ are independently hydrogen, halogen, hydroxy, amino, lower aminoalkyl, trifluoromethyl, lower alkyl, lower alkoxy, lower dialkylamino or lower monoalkylamino.

4. The process of making a compound of formula (4)

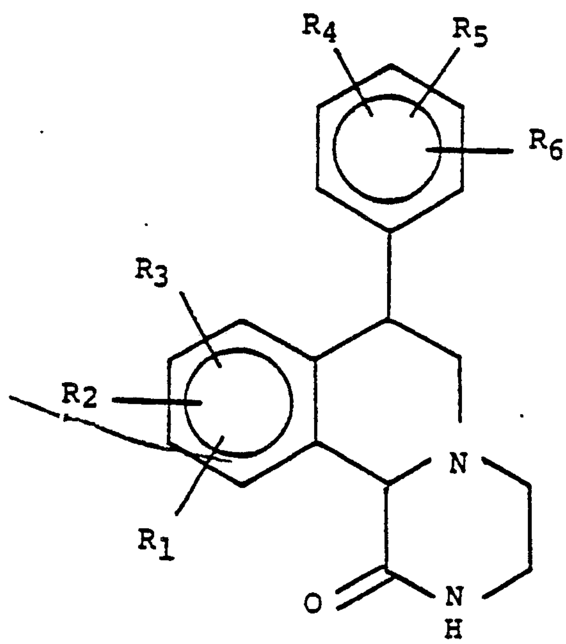

(4)

which comprises reacting a compound of formula (3)

(3)

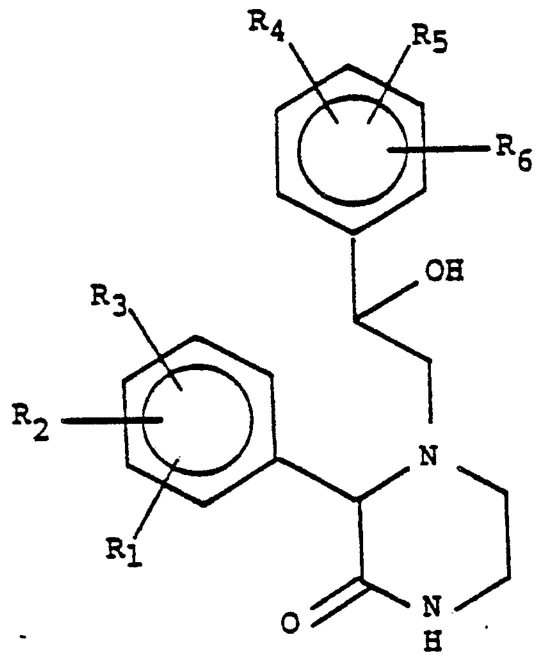

with a strong acid;
wherein
$R_1$, $R_2$, $R_3$, $R_4$, $R_5$ and $R_6$ are independently hydrogen, halogen, hydroxy, amino, lower aminoalkyl, trifluoromethyl, lower alkyl, lower alkoxy, lower dialkylamino or lower monoalkylamino.

5. The process of making a compound of formula (3)

(3)

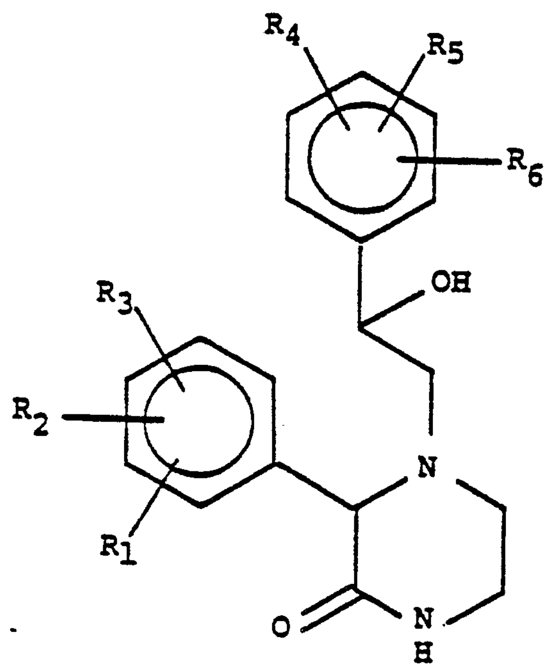

which comprises reacting a compound of formula (2)

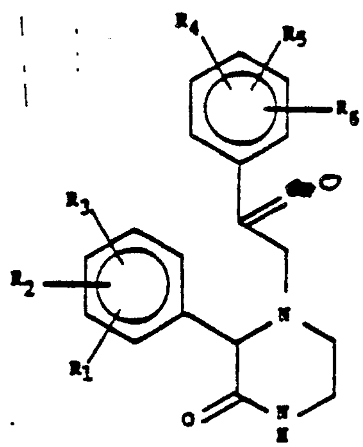

(2)

with a selective reducing agent;
wherein, in compounds (2) and (3)
$R_1$, $R_2$, $R_3$, $R_4$, $R_5$ and $R_6$ are independently hydrogen, halogen, hydroxy, amino, lower aminoalkyl, trifluoromethyl, lower alkyl, lower alkoxy, lower dialkylamino or lower monoalkylamino.

6. A compound of the formula (3)

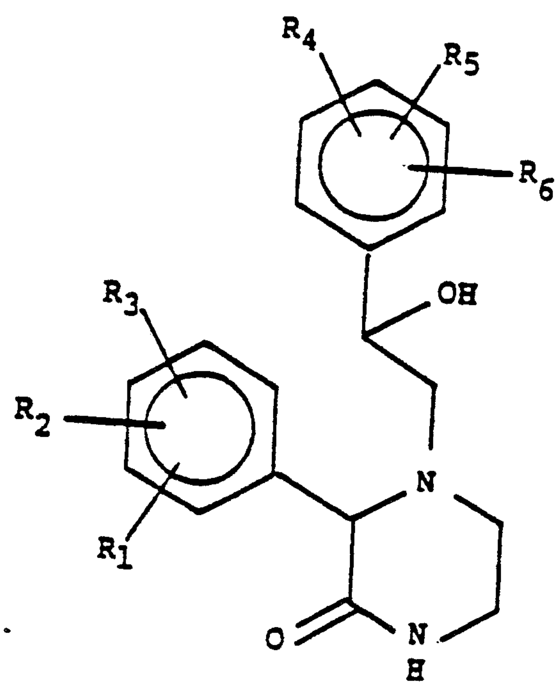

(3)

wherein
$R_1$, $R_2$, $R_3$, $R_4$, $R_5$ and $R_6$ are independently hydrogen, halogen, hydroxy, amino, lower aminoalkyl, trifluoromethyl, lower alkyl, lower alkoxy, lower dialkylamino or lower monoalkylamino.

7. A compound of the formula (4)

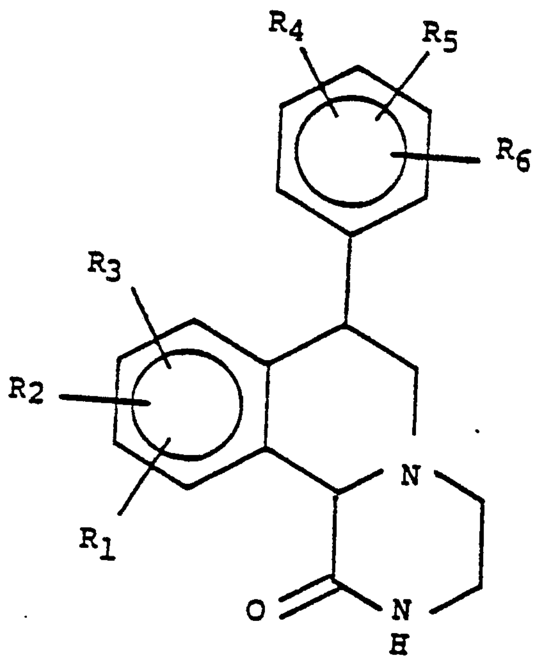

(4)

wherein
$R_1$, $R_2$, $R_3$, $R_4$, $R_5$ and $R_6$ are independently hydrogen, halogen, hydroxy, amino, lower aminoalkyl, trifluoromethyl, lower alkyl, lower alkoxy, lower dialkylamino or lower monoalkylamino.

European Patent Office

# EUROPEAN SEARCH REPORT

Application Number

EP 87 11 0639

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 072 932 (CASSELLA)<br>* Claim 1 *<br>--- | 6 | C 07 D 471/04<br>C 07 D 241/08 //<br>A 61 K 31/495<br>(C 07 D 471/04<br>C 07 D 241:00<br>C 07 D 221:00 ) |
| D,A | EP-A-0 107 825 (PENNWALT)<br>* Claim 18 * & US-A-4 517 187<br>----- | 1 | |
| | | | TECHNICAL FIELDS SEARCHED (Int. Cl.4) |
| | | | C 07 D 471/00<br>C 07 D 241/00 |

The present search report has been drawn up for all claims

| Place of search | Date of completion of the search | Examiner |
|---|---|---|
| THE HAGUE | 18-03-1988 | ALFARO I. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)